(19) Europäisches Patentamt
European Patent Office
Office européen des brevets

(11) Veröffentlichungsnummer: **0 121 183**
**B1**

(12) # EUROPÄISCHE PATENTSCHRIFT

(45) Veröffentlichungstag der Patentschrift: **03.08.88**

(51) Int. Cl.⁴: **C 07 D 249/08**, C 07 D 233/60, A 01 N 43/64, A 01 N 43/50

(21) Anmeldenummer: **84103147.9**

(22) Anmeldetag: **22.03.84**

(54) **Azolylierte Halogen-keten-O,N-acetale, Verfahren zu ihrer Herstellung, diese enthaltende Fungizide und Verfahren zur Bekämpfung von Pilzen mit ihnen.**

(30) Priorität: **29.03.83 DE 3311318**

(43) Veröffentlichungstag der Anmeldung: **10.10.84 Patentblatt 84/41**

(45) Bekanntmachung des Hinweises auf die Patenterteilung: **03.08.88 Patentblatt 88/31**

(84) Benannte Vertragsstaaten: **AT BE CH DE FR GB IT LI NL SE**

(56) Entgegenhaltungen:
**EP - A - 0 056 125**
**EP - A - 0 060 222**
**FR - A - 1 486 817**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Ruland, Alfred, Dr., Schriesheimer Strasse 11, D-6945 Hirschberg (DE)**
Erfinder: **Reuther, Wolfgang, Dr., Am Pferchelhang 16, D-6900 Heidelberg (DE)**
Erfinder: **Pommer, Ernst-Heinrich, Dr., Berliner Platz 7, D-6703 Limburgerhof (DE)**
Erfinder: **Ammermann, Eberhard, Dr., Sachsenstrasse 3, D-6700 Ludwigshafen (DE)**

**EP 0 121 183 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand vorliegender Erfindung sind neue azolylierte Keten-O,N-acetale, ihre Herstellung und Verwendung als Pilzbekämpfungsmittel im Pflanzen- und Materialschutz.

Es ist bekannt, 1-(2'-[2,4-Dichlorphenyl]-2'-[2-propenyloxy]-ethyl)-1H-imidazol (GB-PS 1 318 590) oder das Triphenyl-methyl-1,2,4-triazol (US 3 321 366) als Fungizide zu verwenden. Ihre Wirkung ist jedoch unbefriedigend.

Es wurde nun gefunden, dass azolylierte Halogen-keten-O,N-acetale der allgemeinen Formel

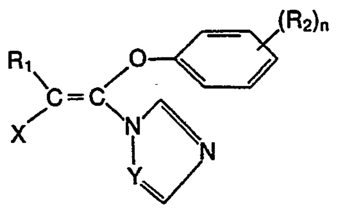

1

in der

$R_1$ gegebenenfalls durch Halogen oder Alkoxy mit 1 bis 4 C-Atomen substituiertes Alkyl mit 1 bis 6 C-Atomen oder Phenyl oder einfach bis dreifach durch Halogen, Alkyl mit 1 bis 6 C-Atomen, Alkoxy mit 1 bis 4 C-Atomen oder Phenyl substituiertes Phenyl,

$R_2$ Alkyl mit 1 bis 6 C-Atomen, Alkoxy mit 1 bis 4 C-Atomen, Halogen, Trifluormethyl, Cyan, Phenyl oder Phenoxy bedeutet, wobei der Phenyl- oder Phenoxyrest gegebenenfalls durch Halogen, Alkyl-($C_1$–$C_4$) oder Alkoxy-($C_1$–$C_4$) substituiert sein kann,

n 0, 1 oder 2,

X Halogen und

Y N oder CH

bedeutet, eine sehr gute fungizide Wirkung haben.

Halogen ist beispielsweise Fluor, Chlor, Brom, Jod, insbesondere Chlor.

Alkyl mit 1 bis 6 C-Atomen ist beispielsweise Methyl, Ethyl, Propyl, iso-Propyl, Butyl, tert.-Butyl, Pentyl.

Alkoxy mit 1 bis 4 C-Atomen ist beispielsweise Methoxy, Ethoxy, iso-Propoxy.

Gegebenenfalls substituiertes Phenyl ist Phenyl oder einfach bis dreifach durch Halogen, Alkyl mit 1 bis 6 C-Atomen, Alkoxy mit 1 bis 4 C-Atomen oder Phenyl substituiertes Phenyl. Salze sind beispielsweise Hydrochloride, Hydrobromide, Sulfate, Phosphate, Acetate, Propionate.

Es wurde ferner gefunden, dass man die Verbindungen der Formel 1 sehr leicht aus den entsprechenden bekannten unsubstituierten Keten-O,N-acetalen, die nach bekannten Verfahren zugänglich sind (DE-A-3 100 261), erhält, wenn man die unsubstituierten Keten-O,N-acetale der Formel 2 bei Temperaturen zwischen −50 und +50 °C in Lösungsmitteln, z.B. Dichlormethan, Chloroform, Tetrachlorkohlenstoff, Tri- oder Tetrachlorethylen mit Halogenen, wie Fluor, Chlor, Brom und Iod zu den dihalogenierten Zwischenverbindungen der Formel 3 umsetzt.

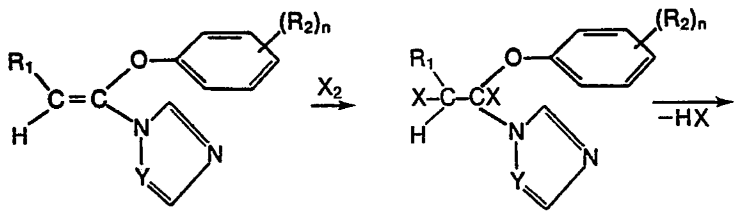


2 3

Die Verbindungen der Formel 3 können teilweise isoliert werden. In der Regel erhält man aber Gemische aus 3 und den entsprechenden HX-Salzen der Produkte 4.

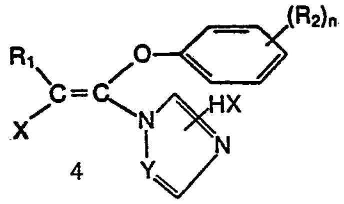

4

Die Verbindungen der Formeln 3 und 4 lassen sich mit Basen wie Alkali- und Erdalkalimetallalkoholaten, vorzugsweise Natriummethylat, aber auch mit Aminen, z. B. Triethylamin, Pyridin, Piperidin bei Temperaturen zwischen 20 und 100 °C, vorzugsweise 20 bis 30 °C, in organischen Lösungsmitteln, wie Alkoholen oder Ethern, vorzugsweise Tetrahydrofuran (THF) oder Dioxan, in Dimethylformamid, Dimethylsulfoxid, Hexamethylphosphorsäuretriamid, in halogenierten Kohlenwasserstoffen wie Methylenchlorid, Tri- oder Tetrachlorethan sowie in Kohlenwasserstoffen wie Benzol oder Toluol nach folgender Gleichung umsetzen.

3 bzw. 4

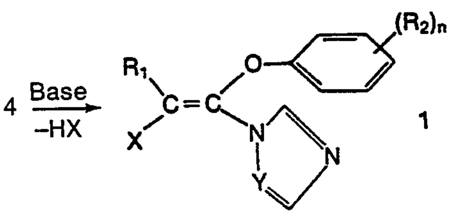


1

In den Formeln 1 bis 4 haben $R_1$, $R_2$, X, Y und n die oben angegebene Bedeutung.

Die Verbindungen der Formel 3 besitzen in der Regel mindestens zwei Chiralitätszentren und liegen als Diastereomerengemische vor. Üblicherweise werden diese Gemische nicht getrennt, sondern als Gemische weiter umgesetzt. Man erhält dann die azolylierten Halogen-keten-O,N-acetale als E/Z-Isomerengemische (Formel 1 E, 1 Z).

Sowohl die reinen Isomeren als auch ihre Gemische werden von der Erfindung umfasst.

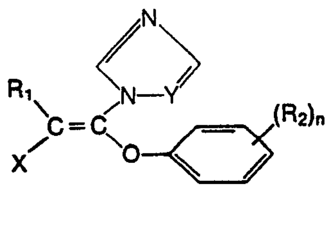

1 Z

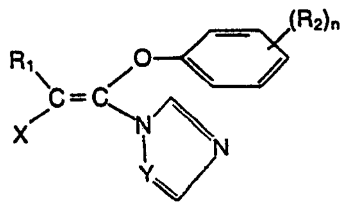

1 E

Die folgenden Beispiele sollen die Herstellung der Verbindungen näher erläutern.

Beispiel 1
1-(Triazol-1-yl)-1-(4-chlorphenoxy)-2-chlor-3,3-dimethyl-but-1-en (Verbindung Nr. 1)

1a) Herstellung der Zwischenverbindung 1-(Triazol-1-yl)-1-(4-chlorphenoxy)-1,2-dichlor-3,3-dimethylbutan

27,7 g (0,1 Mol) 1-(Triazol-1-yl)-(4-chlorphenoxy)-3,3-dimethylbut-1-en werden in 300 ml trokkenem Methylenchlorid gelöst und in die Lösung bei 0 °C so lange Chlor eingeleitet (etwa 2 Minuten), bis mittels Dünnschichtchromatographie keine Ausgangsverbindung mehr nachweisbar ist.

Anschliessend destilliert man das Lösungsmittel im Vakuum ab. Das anfallende ölige Rohprodukt wird ohne weitere Reinigung für die folgende Umsetzung eingesetzt.

$^1$H–NMR (δ-Werte, DDMSO) reines Diastereoisomer

δ = 1.4 (s, 9H); 5.15 (s, 1H); 6.7–7.8 (AA'BB', 4H); 8.45 (s, 1H); 8.95 (s, 1H).

1b) Eliminierung von HCl

34,8 g (0,1 Mol) des gemäss 1a) erhaltenen Dichlorids werden in 200 ml abs. Tetrahydrofuran gelöst und bei −10 °C mit einer Lösung von 12.3 g (0,11 Mol) K-tert.-butanolat in 100 ml abs. Tetrahydrofuran versetzt. Man lässt die Mischung auf 20 °C erwärmen, rührt 30 min nach, hydrolysiert mit Wasser, extrahiert zweimal mit 200 ml Diethylether, trocknet über Natriumsulfat und destilliert das Lösungsmittel im Vakuum ab.

Ausb.: 28 g (90% der Theorie)
Schmp.: 129 °C
$^1$H–NMR (δ-Werte, DDMSO)
δ = 1.35 (s, 9H); 7.1–7.6 (AA'BB, 4H); 8.1 (s, 1H); 9.1 (s, 1H)

Beispiel 2
1-(Triazol-1-yl)-1-(2,4-dichlorphenoxy)-2-brom-3,3-dimethyl-but-1-en (Verbindung Nr. 2)

31,2 g (0,1 Mol) 1-(Triazol-1-yl)-1-(2,4-dichlorphenoxy)-3,3-dimethylbut-1-en werden in 200 ml Methylenchlorid gelöst und mit 16 g (0,1 Mol) Brom in 50 ml Methylenchlorid versetzt. Anschliessend destilliert man das Lösungsmittel ab, gibt 200 ml Diethylether und 150 ml wässrige Natronlauge (50 Gew.%) zu. Man lässt etwa 10 min kräftig rühren, trennt die wässrige Phase ab, wäscht die organische Phase zweimal mit Wasser, trocknet sie über Natriumsulfat und engt im Vakuum ein. Es verbleiben 32.8 g eines viskosen Öls. Ausbeute: 32.8 g (84% der Theorie)

$^1$H–NMR (δ-Werte, $CDCl_3$)

δ = 1.45 (s, 9H); 7.1–7.5 (m, 3H); 8.0 (s, 1H); 8.4 (s, 1H)

Beispiel 3
1-(2,4-Dichlorphenoxy)-1-imidazolyl-2-chlor-3,3-dimethylbut-1-en (Verbindung Nr. 3)

31.3 g (0.1 Mol) 1-(2,4-Dichlorphenoxy)-1-imidazolyl-3,3-dimethylbut-1-en werden in 200 ml Methylenchlorid gelöst und bei 0 °C mit Chlor (etwa 2 Stunden) umgesetzt bis mittels Dünnschichtchromatographie kein Ausgangsprodukt mehr nachweisbar ist. Anschliessend destilliert man das Methylenchlorid im Vakuum ab, gibt 200 ml abs. Tetrahydrofuran zu dem Rückstand und versetzt bei −10 °C mit einer Lösung von 12.3 g (0.11 Mol) K-tert.-butanolat in 100 ml abs. Tetrahydrofuran. Nach dem Erwärmen auf 20 °C hydrolysiert man mit Wasser, extrahiert zweimal mit 200 ml Diethylether, trocknet über Natriumsulfat und destilliert das Lösungsmittel im Vakuum ab.

Ausbeute: 31 g (90% der Theorie)
$^1$H–NMR (δ-Werte, $CDCl_3$)
δ = 1.1 und 1.1 (s, 9H); 6.8–8.1 (m, 6H)

In entsprechender Weise werden die folgenden Verbindungen erhalten.

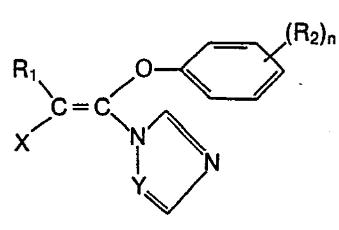

1

| Nr. | $R_1$ | $(R_2)_n$ | X | Y | Schmp. in °C | $^1$H-NMR-Daten δ-Werte in D-DMSO |
|---|---|---|---|---|---|---|
| 3 | $(H_3C)_3C-$ | 2,4–$Cl_2$ | Cl | CH | Öl | E/Z-Isomerengemisch δ = 1.1 und 1,4 (s, 9H); 6.8–8.1 (m, 6H) |
| 4 | $(H_3C)_3C-$ | 2,4–$Cl_2$ | Br | CH | Öl | E/Z-Isomerengemisch δ = 1.2 und 1.4 (s, 9H); 6.8–8.2 (m, 6H) |
| 5 | $(H_3C)_3C-$ | 2–Cl | Cl | CH | Öl | δ = 1.4 (s, 9H); 6.8–8.2 (m, 7H) |
| 6 | $(H_3C)_3C-$ | 2–Cl | Br | CH | Öl | δ = 1.2 (s, 9H); 6.8–8.2 (m, 7H) |
| 2 | $(H_3C)_3C-$ | 2,4–$Cl_2$ | Br | N | Öl | δ = 1.45 (s, 9H); 7.1–7.5 (m, 3H); 8.0 (s, 1H); 8.4 (s. 1H) |
| 7 | $(H_3C)_3C-$ | 2,4–$Cl_2$ | Cl | N | Öl | δ = 1.45 (s, 9H); 7.1–7.5 (s, 3H); 8.0 (s, 1H); 8.4 (s. 1H) |
| 1 | $(H_3C)_3C-$ | 4–Cl | Cl | N | 129 | δ = 1.35 (s, 9H); 7.1–7.6 (AA'BB', 4H); 8.1 (s, 1H); 9.15 (s, 1H) |
| 8 | $(H_3C)_3C-$ | 4-Phenyl | Cl | N | Öl | δ = 1.4 (s, 9H); 7.1–7.8 (m, 9H); 8.1 (s, 1H); 9.15 (s. 1H) |
| 9 | $(H_3C)_3C-$ | 4-Phenyl | Br | N | Öl | δ = 1.45 (s, 9H); 6.9–7.7 (m, 9H); 7.9 (s, 1H) 8.5 (s, 1H) |
| 10 | $(H_3C)_3C-$ | 4–Br | Cl | N | Öl | δ = 1.35 (s, 9H); 7.1–7.7 (AA'BB', 4H); 8.1 (s, 1H); 9.1 (s, 1H) |
| 11 | $(H_3C)_3C-$ | 4–Br | Br | N | Öl | δ = 1.35 (s, 9H); 7–7.6 (AA'BB', 4H); 8.1 (s, 1H); 9.1 (s, 1H) |

Die neuen Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, sowie Gemüse, wie Gurken, Bohnen und Kürbisgewächse.

Die neuen Verbindungen sind insbesondere geeignet zur Bekämpfung folgender Pflanzenkrankheiten:

Erysiphe graminis (echter Mehltau) in Getreide,
Erysiphe cichoracearum an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Phythophthora infestans (falscher Mehltau) an Kartoffeln, Tomaten,
Pyricularia oryzae an Reis,
Pseudocercosporella herpotrichoides an Weizen.

Ausserdem besitzen die Wirkstoffe eine vorteilhafte mikrozide Wirksamkeit zum Beispiel gegen Candida albicans und Trichophyton mentagrophytes.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwekken; sie sollen in jedem Fall eine feine und gleichmässige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle, z.B. Kaoline, Tonerden, Talkum, Kreide und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkyl-sulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,1 und 3 kg Wirkstoff oder mehr je ha.

Die neuen Verbindungen können auch im Materialschutz eingesetzt werden. So lassen sich mit den erfindungsgemässen Holzschutzmitteln beispielsweise folgende holz- und anstrichverfärbende Pilze, Moderfäulepilze und holzzerstörende Pilze bekämpfen: Pullularia (Aureobasidium pullulans), Sclerophoma pityophila, Ceratocystis spec., Paecilomyces variotii, Hormiscium spec., Stemphylium spec., Phoma violacea, Cladosporium herbarum, Trichoderma viride, Chaetomium globosum, Humicola grisea, Merulius lacrimans, Coniophora puteana, Lentinus lepideus, Lenzites trabea, Polystictus versicolor, Stereum hirsutum, Fomes annosus.

Die neuen Wirkstoffe können in Zubereitungen, wie Lösungen, Emulsionen, Pasten und Öldispersionen, angewendet werden. Die Zubereitungen enthalten im allgemeinen zwischen 0,1 und 90 Gew.% Wirkstoff, vorzugsweise 0,25 bis 50%. Die Aufwandmengen betragen je nach Art des gewünschten Effektes 0,5 bis 8 g Wirkstoff je $m^2$ zu schützender Holzoberfläche bzw. 50 bis 4000 g Wirkstoff/$m^3$ Holz. Anstrichfarben enthalten 0,5 bis 5 Gew.% Wirkstoff. Zum Schutz von Holzwerkstoffen werden die Wirkstoffe als Emulsion oder im Untermischverfahren dem Klebestoff in Mengen von 2 bis 6 Gew.% zugesetzt. Die Anwendung der Wirkstoffe erfolgt durch Streichen, Spritzen, Sprühen, Tauchen oder Druckimprägnierungs- oder Diffusionsverfahren. Zur Herstellung wasserabweisender Imprägnieranstriche können den öligen Holzschutzmitteln sog. «water reppellents» zugesetzt werden. Geeignete Substanzen sind beispielsweise Zinkstearat, Aluminiumstearat, Wachse. Ferner können zur Erzielung von Farbeffekten anorganische oder organische Pigmente in die Formulierungen eingearbeitet werden.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Giessen.

I. 20 Gewichtsteile der Verbindung des Beispiels 2 werden in 3 Gewichtsteilen des Natriumsalzes der Diisobutylnaphthalin-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in Wasser erhält man eine Spritzbrühe.

II. 3 Gewichtsteile der Verbindung des Beispiels 6 werden mit 97 Gewichtsteilen feinteiligem Kaolin innig vermischt. Man erhält auf diese Weise ein Stäubemittel, das 3 Gew.% des Wirkstoffs enthält.

III. 30 Gewichtsteile der Verbindung des Beispiels 8 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV. 40 Gewichtsteile der Verbindung des Beispiels 9 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wässrige Dispersion. Durch Verdünnen mit Wasser erhält man eine wässrige Dispersion.

V. 20 Teile der Verbindung des Beispiels 3 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

VI. Man vermischt 90 Gewichtsteile der Verbindung des Beispiels 10 mit 10 Gewichtsteilen N-Methyl-alpha-pyrrolidon und erhält eine Lösung, die zur Anwendung in Form kleinster Tropfen geeignet ist.

VII. 20 Gewichtsteile der Verbindung des Beispiels 4 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgiessen und feines Verteilen der Lösung in Wasser erhält man eine wässrige Dispersion.

VIII. 20 Gewichtsteile der Verbindung des Beispiels 4 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingiessen und feines Verteilen der Lösung in Wasser erhält man eine wässrige Dispersion.

IX. 10 Gewichtsteile der Verbindung des Beispiels 3, 20 Gewichtsteile Polyoxyethylensorbitanmonolaurat, 20 Gewichtsteile Methanol und 50 Gewichtsteile Wasser werden zu einer Lösung verrührt, die 10 Gew.% des Wirkstoffs enthält. Durch Hinzufügen von weiterem Wasser können stärker verdünnte Lösungen hergestellt werden.

X. Zur Herstellung eines lösemittelhaltigen Holzschutzmittels mit 1% Wirkstoff wird zunächst 1 Teil (Gew.-Teil) der Verbindung 9 unter leichtem Erwärmen in 55 Teilen einer aromatenreichen Benzinfraktion gelöst. Anschliessend werden 10 Teile eines Alkydharzes zugefügt und bei Raumtemperatur mit Testbenzin auf 100 Teile ergänzt.

In entsprechender Weise können lösemittelhaltige Holzschutzmittel bis 5 Gew.% des Wirkstoffs hergestellt werden.

Die neuen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrösserung des Wirkungsspektrums. Bei einer Anzahl solcher

Fungizidmischungen treten auch synergistische Effekte auf, d.h. die Wirksamkeit des Kombinationsproduktes ist grösser als die addierten Wirksamkeiten der Einzelkomponenten.

Fungizide, die mit den neuen Verbindungen kombiniert werden können, sind beispielsweise:
Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat)
Ammoniak-Komplex von Zink-(N,N-propylen-bis-dithiocarbamat),
Zink-(N,N′-propylen-bis-dithiocarbamat),
N,N′-Propylen-bis(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat,
5-Nitro-isophthalsäure-di-isopropylester,
heterocyclische Substanzen, wie
2-Heptadecyl-2-imidazolin-acetat,
2,4-Dichlor-6-(o-chloranilino)-s-triazin,
0,0-Diethyl-phthalimidophosphonothioat,
5-Amino-1-(bis-(dimethylamino)-phosphinyl)-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Thiazolyl-(4))-benzimidazol,
2-(Furyl-(2)-benzimidazol,
N-(1,1,2,2-Tetrachlorethylthio)-tetrahydrophthalimid,
N-Trichlormethylthio-tetrahydrophthalimid,
N-Trichlormethyl-phthalimid,
N-Dichlorfluormethylthio-N′,N′-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid
2-Methyl-5,6-dihydro-4H-pyran-3-carbonsäure-anilid
2-Methyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid,
2-Iod-benzoesäure-anilid,
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze,
2,6-Dimethyl-N-cyclododecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol,
1[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N′-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
alpha-(2-Chlorphenyl)-alpha-(4-chlorphenyl)-5-pyrimidin-methanol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol
sowie verschiedene Fungizide, wie
Dodecylguanidinacetat,
3-(3-(3,5-Dimethyl-2-oxycyclohexyl)-2-hydroxyethyl)-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2′-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin,
3-(3,5-Dichlorphenyl)-5-methyl-5-methoxymethyl-1,3-oxazolidin-2,4-dion
3-(3,5-Dichlorphenyl)-1-isopropylcarbamoyl-hydantoin
N-(3,5-Dichlorphenyl)-1,2-dimethylcyclopropan-1,2-dicarbonsäureimid.

Zur Vergrösserung des Wirkungsspektrums insbesondere zur Bekämpfung holzzerstörender bzw. holz- oder anstrichverfärbender Pilze können die Wirkstoffe mit folgenden Wirkstoffen kombiniert werden:

Organozinnverbindungen, wie Tributylzinnoxid und Tributylzinnbenzoat
Methylenbisthiocyanat
Alkyl-dimethyl-benzylammoniumchlorid
Cetyl-pyridiniumchlorid
Chlorierte Phenole, wie Tetra- und Pentachlorphenol
Tetrachlorisophthalsäure-dinitril
2-Halogenbenzoesäureanilid

N-Cyclohexyl-N-methoxy-2,5-dimethyl-furan-3-carbonsäureamid
N,N-Dimethyl-N'-phenyl-(N'-fluordicyclomethylthio)-sulfamid
N-Phenyl-N,N'-dimethyl-N'-fluordichlormethylthiosulfonyl-diamid
Benzimidazol-2-carbaminsäure-methylester
2-Thiocyanomethyl-thiobenzothiazol
Kupfernaphthenat
Kupfer-8-oxychinolin
Alkali- und Metallsalze des N'-Hydroxy-N-cyclohexyl-diazeniumoxids.

Zur Prüfung der fungiziden Wirksamkeit der neuen Verbindungen wurden die folgenden Versuche durchgeführt. Hierbei wurden zu Vergleichszwecken die folgenden bekannten Wirkstoffe verwendet.

Triphenyl-methyl-1,2,4-triazol A
(bekannt aus US 3 321 366)
1-(2'-[2,4-Dichlorphenyl]-2'-[2-propenyloxy]-ethyl)-1H-imidazol B
(bekannt aus GB-1 318 590).

Versuch 1
Wirksamkeit gegen Weizenmehltau

Blätter von in Töpfen gewachsenen Weizenkeimlingen der Sorte «Jubilar» werden mit wässriger Spritzbrühe, die 80% Wirkstoff und 20% Emulgiermittel in der Trockensubstanz enthält, besprüht und 24 Stunden nach dem Antrocknen des Spritzbelages mit Oidien (Sporen) des Weizenmehltaus (Erysiphe graminis var. tritici) bestäubt. Die Versuchspflanzen werden anschliessend im Gewächshaus bei Temperaturen zwischen 20 und 22 °C und 75 bis 80% relativer Luftfeuchtigkeit aufgestellt. Nach 7 Tagen wird das Ausmass der Mehltauentwicklung ermittelt.

Das Ergebnis des Versuchs zeigt, dass beispielsweise die Verbindungen 1, 2, 3, 7, 8, 9, 10 und 11 bei Anwendung als 0,025; 0,006 oder 0,0015%ige (gew.%ige) Spritzbrühe eine bessere fungizide Wirkung (100%) zeigen als die Wirkstoffe A und B (70%).

Versuch 2
Wirksamkeit gegen Weizenbraunrost

Blätter von in Töpfen gewachsenen Weizensämlingen der Sorte «Jubilar» werden mit Sporen des Braunrostes (Puccinia recondita) bestäubt. Danach werden die Töpfe für 24 Stunden bei 20 bis 22 °C in eine Kammer mit hoher Luftfeuchtigkeit (90 bis 95%) gestellt. Während dieser Zeit keimen die Sporen aus und die Keimschläuche dringen in das Blattgewebe ein. Die infizierten Pflanzen werden anschliessend mit wässrigen Spritzbrühen, die 80% Wirkstoff und 20% Emulgiermittel in der Trockensubstanz enthalten, tropfnass gespritzt. Nach dem Antrocknen des Spritzbelages werden die Versuchspflanzen im Gewächshaus bei Temperaturen zwischen 20 und 22 °C und 65 bis 70% relaltiver Luftfeuchte aufgestellt. Nach 8 Tagen wird das Ausmass der Rostpilzentwicklung auf den Blättern ermittelt.

Das Ergebnis des Versuches zeigt, dass beispielsweise die Verbindungen Nr. 1, 2, 3, 7, 9 und 11 bei der Anwendung als 0,025%ige Spritzbrühe eine bessere fungizide Wirkung (z.B. 97%) zeigen als der Wirkstoff A (50%).

Versuch 3
Wirksamkeit gegen Botrytis cinerea an Paprika

Paprikasämlinge der Sorte «Neusiedler Ideal Elite» werden, nachdem sich 4 bis 5 Blätter gut entwickelt haben, mit wässrigen Suspensionen, die 80% Wirkstoff und 20% Emulgiermittel in der Trockensubstanz enthalten, tropfnass gespritzt. Nach dem Antrocknen des Spritzbelages werden die Pflanzen mit einer Konidienaufschwemmung des Pilzes Botrytis cinerea besprüht und bei 22 bis 24 °C in eine Kammer mit hoher Luftfeuchtigkeit gestellt. Nach 5 Tagen hat sich die Krankheit auf den unbehandelten Kontrollpflanzen so stark entwickelt, dass die entstandenen Blattnekrosen den überwiegenden Teil der Blätter bedecken.

Das Ergebnis des Versuches zeigt, dass beispielsweise die Verbindungen Nr. 1, 2, 3 und 7 bei der Anwendung als 0,05%ige Spritzbrühe eine bessere fungizide Wirkung zeigen (z.B. 97%) als der Wirkstoff A (10%).

**Patentansprüche**

1. Azolylierte Halogen-keten-O,N-acetale der allgemeinen Formel

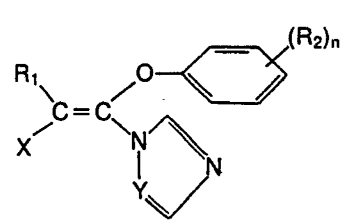

1

in der

$R_1$ gegebenenfalls durch Halogen oder Alkoxy mit 1 bis 4 C-Atomen substituiertes Alkyl mit 1 bis 6 C-Atomen oder Phenyl oder einfach bis dreifach durch Halogen, Alkyl mit 1 bis 6 C-Atomen, Alkoxy mit 1 bis 4 C-Atomen oder Phenyl substituiertes Phenyl,

$R_2$ Alkyl mit 1 bis 6 C-Atomen, Alkoxy mit 1 bis 4 C-Atomen, Halogen, Trifluormethyl, Cyan, Phenyl oder Phenoxy bedeutet, wobei der Phenyl- oder Phenoxyrest gegebenenfalls durch Halogen, Alkyl-($C_1$–$C_4$) oder Alkoxy-($C_1$–$C_4$) substituiert sein kann,

n 0, 1 oder 2,

X Halogen und

Y N oder CH

bedeutet und ihre Salze.

2. Verfahren zur Herstellung von Verbindungen gemäss Anspruch 1, dadurch gekennzeichnet, dass man azolylierte Keten-O,N-acetale der Formel 2, in der $R^1$, $R^2$, Y und n die im Anspruch 1 genannten Bedeutungen haben, bei Temperaturen zwischen −50 und +50 °C in Lösungsmitteln mit Halogenen (X) umsetzt und die entstehenden Additionsverbindungen 3 zu den Verbindungen der Formel 1 umsetzt.

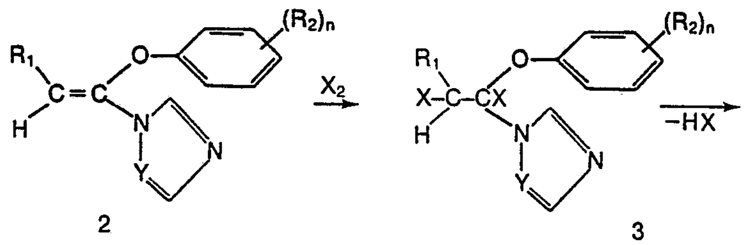


1

2 3

3. Fungizide Mittel, gekennzeichnet durch einen Gehalt an einem azolylierten Halogen-keten-O,N-acetal gemäss Anspruch 1.

4. Verfahren zur Bekämpfung von Pilzen, dadurch gekennzeichnet, dass man die Pilze oder die vor Pilzbefall zu schützenden Materialien, Pflanzen, Saatgüter oder den Boden mit einer Verbindung gemäss Anspruch 1 behandelt.

5. 1-(Triazol-1-yl)-1-(4-chlorphenoxy)-2-chlor-3,3-dimethyl-but-1-en.

6. 1-(Triazol-1-yl)-1-(2,4-dichlorphenoxy)-2-brom-3,3-dimethyl-but-1-en.

7. 1-(Imidazol-1-yl)-1-(2,4-dichlorphenoxy)-2-chlor-3,3-dimethyl-but-1-en.

8. 1-(Triazol-1-yl)-1-(2,4-dichlorphenoxy)-2-chlor-3,3-dimethyl-but-1-en.

9. Fungizide Mittel, gekennzeichnet durch einen Gehalt an einem azolylierten Halogen-keten-O,N-acetal der allgemeinen Formel

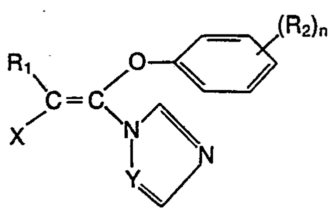

1

in der

$R_1$ gegebenenfalls durch Halogen oder Alkoxy mit 1 bis 4 C-Atomen substituiertes Alkyl mit 1 bis 6 C-Atomen oder Phenyl oder einfach bis dreifach durch Halogen, Alkyl mit 1 bis 6 C-Atomen, Alkoxy mit 1 bis 4 C-Atomen oder Phenyl substituiertes Phenyl,

$R_2$ Alkyl mit 1 bis 6 C-Atomen, Alkoxy mit 1 bis 4 C-Atomen, Halogen, Trifluormethyl, Cyan, Phenyl oder Phenoxy bedeutet, wobei der Phenyl- oder Phenoxyrest gegebenenfalls durch Halogen, Alkyl-($C_1$–$C_4$) oder Alkoxy-($C_1$–$C_4$) substituiert sein kann,

n 0, 1 oder 2,

X Halogen und

Y N oder CH

bedeutet oder seinen Salzen und einem Trägerstoff.

## Claims

1. An azolyl-substituted haloketene O,N-acetal of the formula

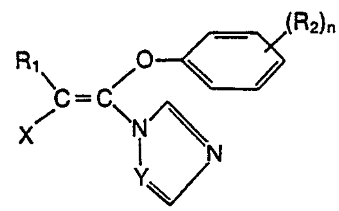

1

where $R_1$ is alkyl of 1 to 6 carbon atoms which is unsubstituted or substituted by halogen or by alkoxy of 1 to 4 carbon atoms, or is phenyl which is unsubstituted or monosubstituted to trisubstituted by halogen, alkyl of 1 to 6 carbon atoms, alkoxy of 1 to 4 carbon atoms or phenyl, $R_2$ is alkyl of 1 to 6 carbon atoms, alkoxy of 1 to 4 carbon atoms, halogen, trifluoromethyl, cyano, phenyl or phenoxy, where the phenyl or phenoxy radical may be unsubstituted or substituted by halogen, $C_1$–$C_4$-alkyl or $C_1$–$C_4$-alkoxy, n is 0, 1 or 2, X is halogen and Y is N or CH, and its salts.

2. A process for the preparation of a compound as claimed in claim 1, wherein an azolyl-substituted ketene O,N-acetal of the formula 2, where $R^1$, $R^2$ [sic], Y and n have the meanings stated in claim 1, is reacted with a halogen (X) at from −50 to +50 °C in a solvent, and the resulting adduct 3 is converted to a compound of the formula 1.

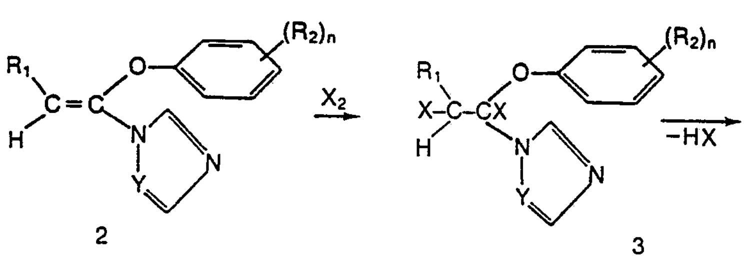


1

2 3

3. A fungicide which contains an azolyl-substituted haloketene O,N-acetal as claimed in claim 1.

4. A method of controlling fungi, wherein the fungi or the materials, plants or seed to be protected from fungal attack, or the soil, are or is

treated with a compound as claimed in claim 1.

5. 1-(Triazol-1-yl)-1-(4-chlorophenoxy)-2-chloro-3,3-dimethyl-but-1-ene.

6. 1-(Triazol-1-yl)-1-(2,4-dichlorophenoxy)-2-bromo-3,3-dimethyl-but-1-ene.

7. 1-(Imidazol-1-yl)-1-(2,4-dichlorophenoxy)-2-chloro-3,3-dimethyl-but-1-ene.

8. 1-(Triazol-1-yl)-1-(2,4-dichlorophenoxy-2-chloro-3,3-dimethyl-but-1-ene.

9. A fungicide which contains an azolyl-substituted haloketene O,N-acetal of the formula

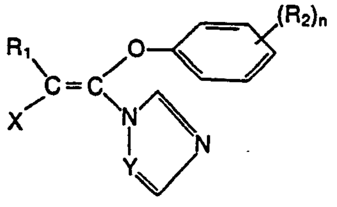

1

where $R_1$ is alkyl of 1 to 6 carbon atoms which is unsubstituted or substituted by halogen or by alkoxy of 1 to 4 carbon atoms, or is phenyl which is unsubstituted or monosubstituted to trisubstituted by halogen, alkyl of 1 to 6 carbon atoms, alkoxy of 1 to 4 carbon atoms or phenyl, $R_2$ is alkyl of 1 to 6 carbon atoms, alkoxy of 1 to 4 carbon atoms, halogen, trifluoromethyl, cyano, phenyl or phenoxy, where the phenyl or phenoxy radical may be unsubstituted or substituted by halogen, $C_1$–$C_4$-alkyl or $C_1$–$C_4$-alkoxy, n is 0, 1 or 2, X is halogen and Y is N or CH, or its salts and a carrier.

**Revendications**

1. Halogéno-cétène-O,N-acétals azolés de la formule générale

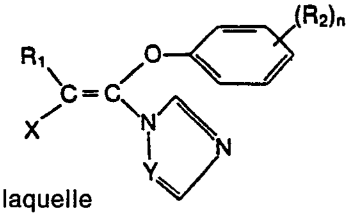

1

dans laquelle

$R_1$ représente un radical alkyle comportant de 1 à 6 atomes de carbone à substitution halogénée ou alcoxylique en $C_1$ à $C_4$ éventuelle, ou un radical phényle, ou un radical phényle substitué de une à trois fois par des atomes d'halogènes, des radicaux alkyle comportant de 1 à 6 atomes de carbone, des radicaux alcoxy comportant de 1 à 4 atomes de carbone, ou des radicaux phényle,

$R_2$ représente un radical alkyle comportant de 1 à 6 atomes de carbone, alcoxy comportant de 1 à 4 atomes de carbone, des atomes d'halogènes, des radicaux trifluorométhyle, cyano, phényle ou phénoxy, où le radical phényle ou phénoxy peut être à substitution halogénée, alkylique en $C_1$–$C_4$ ou alcoxylique en $C_1$–$C_4$ éventuelle,

n est égal à 0, 1 ou 2,

X représente un atome d'halogène et Y représente un atome d'azote ou le groupe CH

et leurs sels.

2. Procédé de préparation de composés suivant la revendication 1, caractérisé en ce que l'on fait réagir des cétène-O,N-acétals azolylés de la formule 2, dans laquelle $R^1$, $R^2$, Y et n ont les significations qui leur ont été précédemment attribuées dans la revendication 1, à des températures qui fluctuent de $-50$ à $+50\,°C$, dans des solvants, avec des halogènes (X) et on convertit les composés d'addition 3 engendrés en les composés de la formule 1

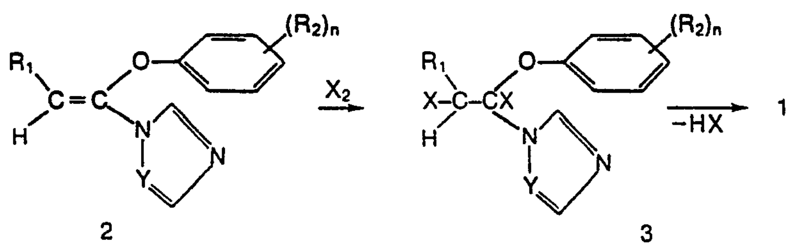


2 3

3. Agents fongicides, caractérisés par une teneur en un halogéno-cétène-O,N-acétal azolé suivant la revendication 1.

4. Procédé de lutte contre des champignons, caractérisé en ce que l'on traite les champignons, ou le sol, les graines ou semences, les plantes ou les matériaux, à protéger préalablement à l'attaque des champignons, par un composé suivant la revendication 1.

5. 1-(Triazole-1-yl)-1-(4-chlorophénoxy)-2-chloro-3,3-diméthyl-but-1-ène.

6. 1-(Triazole-1-yl)-1-(2,4-dichlorophénoxy)-2-bromo-3,3-diméthyl-but-1-ène.

7. 1-(Imidazole-1-yl)-1-(2,4-dichlorophénoxy)-2-chloro-3,3-diméthyl-but-1-ène.

8. 1-(Triazole-1-yl)-1-(2,4-dichlorophénoxy)-2-chloro-3,3-diméthyl-but-1-ène.

9. Agents fongicides, caractérisés par une teneur en un halogèno-cétène-O,N-acétal azolé de la formule générale

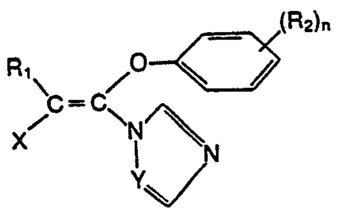

1

dans laquelle

$R_1$ représente un radical alkyle comportant de 1 à 6 atomes de carbone à substitution halogénée ou alcoxylique en $C_1$ à $C_4$ éventuelle, ou un radical phényle, ou un radical phényle substitué de une à trois fois par des atomes d'halogènes, des radicaux alkyle comportant de 1 à 6 atomes de carbone, des radicaux alcoxy comportant de 1 à 4

atomes de carbone ou des radicaux phényle,

$R_2$ représente un radical alkyle comportant de 1 à 6 atomes de carbone, alcoxy comportant de 1 à 4 atomes de carbone, des atomes d'halogènes, des radicaux trifluorométhyle, cyano, phényle ou phénoxy, où le radical phényle ou phénoxy peut être à substitution halogénée, alkylique en $C_1$–$C_4$ ou alcoxylique en $C_1$–$C_4$ éventuelle,

n est égal à 0, 1 ou 2,

X représente un atome d'halogène et Y représente un atome d'azote ou le groupe CH ou ses sels et un véhicule ou support.